(19) Europäisches Patentamt / European Patent Office / Office européen des brevets

(11) **EP 2 556 489 B1**

(12) # EUROPÄISCHE PATENTSCHRIFT

(45) Veröffentlichungstag und Bekanntmachung des Hinweises auf die Patenterteilung:
**29.01.2014 Patentblatt 2014/05**

(21) Anmeldenummer: **11720001.4**

(22) Anmeldetag: **07.04.2011**

(51) Int Cl.:
**G06T 5/00** *(2006.01)*     **G06T 11/00** *(2006.01)*

(86) Internationale Anmeldenummer:
**PCT/CH2011/000074**

(87) Internationale Veröffentlichungsnummer:
**WO 2011/123964 (13.10.2011 Gazette 2011/41)**

(54) **ABGLEICH VON POSITRONEN EMISSIONS TOMOGRAPHEN UNTER VERWENDUNG EINES VIRTUELLEN TOMOGRAPHEN**

ALIGNMENT OF POSITRON EMISSION TOMOGRAPHS USING A VIRTUAL TOMOGRAPH

ALIGNEMENT DE TOMOGRAPHES PAR ÉMISSION DE POSITRONS EN UTILISANT UN TOMOGRAPHE VIRTUEL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.04.2010 CH 505102010**

(43) Veröffentlichungstag der Anmeldung:
**13.02.2013 Patentblatt 2013/07**

(73) Patentinhaber:
 • **Universität Bern**
   **3012 Bern (CH)**
 • **Universitätsklinik Für Nuklearmedizin**
   **3010 Bern (CH)**

(72) Erfinder: **WEITZEL, Thilo**
   **CH-3037 Herrenschwanden (CH)**

(74) Vertreter: **Herrmann, Uwe**
   **Lorenz - Seidler - Gossel**
   **Widenmayerstrasse 23**
   **80538 München (DE)**

(56) Entgegenhaltungen:
   **US-A1- 2006 054 827**

 • **OLIVIER ROUSSET ET AL: "Partial Volume Correction Strategies in PET", PET CLINICS, WB SAUNDERS CO, US, Bd. 2, Nr. 2, 1. April 2007 (2007-04-01), Seiten 235-249, XP002525044, ISSN: 1556-8598, DOI: DOI:10.1016/J.CPET.2007.10.005 [gefunden am 2008-02-15]**
 • **M. SORET ET AL: "Partial-Volume Effect in PET Tumor Imaging", THE JOURNAL OF NUCLEAR MEDICINE, Bd. 48, Nr. 6, 1. Juni 2007 (2007-06-01), Seiten 932-945, XP55001511, ISSN: 0161-5505, DOI: 10.2967/jnumed.106.035774 in der Anmeldung erwähnt**

**Beschreibung**

Die Positronen-Emissions-Tomographie (PET), gegebenenfalls in Kombination mit Computer-Tomographie (PET/CT), erlaubt neben der medizinisch diagnostischen Bildgebung prinzipiell auch die quantitative Bestimmung verschiedener Parameter. Voraussetzungen für derartige quantitative Messungen am Patienten sind die strikte Einhaltung standardisierter Untersuchungsprotokolle sowie eine Eichung bzw. ein Abgleich der verwendeten Tomographen. Insbesondere im Kontext multi zentrischer medizinischer Studien werden derzeit die Anforderungen an einheitliche Untersuchungsprotokolle, an den Abgleich der Tomographen und an die Methoden zur Bestimmung quantitativer Werte intensiv diskutiert [WAHL09], [BOEL09], [BOEL09b], [DELB06], [SHYA08], [SCHE09].

Quantitative Messungen unter Verwendung von Tomographen zeigen jedoch eine starke und unvermeidliche Abhängigkeit von den Abbildungseigenschaften des jeweiligen Tomographen, insbesondere der räumlichen Transferfunktion (Point Spread Function, PSF), welche sich von Tomograph zu Tomograph unterscheidet. Selbst bei ansonsten geeichten Tomographen oder geeichten anderen medizinischen bildgebenden Geräten wird infolge der unterschiedlichen Transferfunktionen eine quantitative Bewertung erschwert. Dies betrifft insbesondere multi-zentrische medizinische Studien, welche eine quantitative Vergleichbarkeit der von den teilnehmenden Zentren gemessenen Daten zwingend voraussetzen.

Das im Kontext der Positronen Emissions Tomographie am Häufigsten verwendete quantitative Mass für die in einem Gewebe gemessenen Aktivität ist der "Standardized Uptake Value" (SUV) in unterschiedlichen Varianten [BOEL09]. Es wurde gezeigt, dass die Bestimmung eines SUV mit grosser Wiederholgenauigkeit durchgeführt werden kann, wenn ein strikt definiertes Untersuchungsprotokoll eingehalten wird und jeweils derselbe Tomograph verwendet wird.

Gerade diese Messungen fallen jedoch gegebenenfalls sehr unterschiedlich aus, wenn unterschiedliche Tomographen verwendet werden, und zwar auch dann, wenn die Geräte geeicht sind. Ursache ist vor allem der Partial Volumen Effekt (PVE), der bewirkt, dass die Aktivität kleinerer Objekte systematisch unterschätzt wird. Dieser Effekt steht in engem Zusammenhang mit der räumlichen Transferfunktion des jeweiligen Tomographen. Neben der durch die Halbwertsbreite der Transferfunktion bestimmten räumlichen Auflösung spielt hier insbesondere auch die genaue Form der Transferfunktion eine Rolle.

Unterschiedliche Tomographen können somit selbst bei gleicher räumlicher Auflösung sehr unterschiedliche Partial Volumen Effekte (PVE) zeigen.

Es wurden unterschiedliche Methoden vorgeschlagen, um den Einfluss des PVE auf quantitative Messungen insbesondere Messungen des SUV zu minimieren [KEYE95] [STRA91] [BOEL03] [WEST06] [SORE07].

In dem Artikel Oliver Rousset et al.: "Partial Volume Correction Strategies in PET", PET Clin 2 (2007), 235 - 249 (Elsevier Saunders) werden unter Bezugnahme auf einen umfangreichen Stand der Technik unterschiedliche Methoden zur Partial Volumen Korrektur zusammengefasst. Die vorgestellten Verfahren umfassen die "postreconstruction" sowie die "reconstruction" basierte PVE-Korrektur. Der Artikel offenbart den beschriebenen Stand der Technik im Hinblick auf die Handhabung des PVE im Sinne einer Korrektur der Messung, wobei die dargestellten Methoden sehr unterschiedliche Eigenschaften haben und zu unterschiedlichen Ergebnissen führen können. Durch die dargestellten Messverfahren ergibt sich im Wesentlichen eine Verbesserung der Ergebnisse. Unter anderem wird eine iterative Entfaltung der gemessenen Aktivitätsverteilung mittels einer scannerspezifischen Punktabbildungsfunktion (PSF) offenbart. Beschrieben wird weiterhin, dass auf Deconvolution basierende Verfahren unter Nutzung der PSF nicht oder nur sehr begrenzt eingesetzt werden können, da das resultierende Rauschen die Ergebnisse untauglich macht.

Mit Hilfe dieser oft aufwändigen Methoden, gegebenenfalls unter Verwendung unabhängiger Messungen zur Bestimmung des Volumens eines Objektes, gelingt es zwar die Genauigkeit der Messungen zu erhöhen oder den verfügbaren Messbereich zu kleineren Objekten hin auszudehnen, die Ergebnisse bleiben aber trotzdem abhängig vom jeweils verwendeten Tomographen.

Der Nutzen dieser Verfahren im Kontext multi zentrischer Studien bleibt somit sehr begrenzt.

Im Kontext von quantitativen PET Untersuchungen, insbesondere solchen, die mehrfach über einen längeren Zeitraum oder an verschiedenen Orten durchgeführt werden müssen, wäre ein standardisierter Tomograph erforderlich, welcher jederzeit und überall reproduzierbare Messungen liefern könnte.

Ein derartiges standardisiertes PET System steht leider nicht zur Verfügung, einerseits in Folge der Vielzahl verfügbarer unterschiedlicher Systeme verschiedner Hersteller andererseits in Folge der ständigen technischen Weiterentwicklung der Systeme und den dadurch bedingten ständigen Wechsel der Geräte.

**Zielsetzung der Erfindung und Lösung**

Der wesentliche Aspekt quantitativer Messungen ist die Möglichkeit, Messungen miteinander zu vergleichen. Im Kontext der PET kann eine Messung des SUV erfolgen als Grundlage einer Therapie-Kontrolle (Vergleich mehrerer Messungen über die Zeit), als Grundlage einer Klassifizierung (Vergleich von Messungen an mehreren Patienten) oder etwa im Rahmen einer medizinischen Studie (Verlaufskontrolle oder Klassifizierung bei einer Vielzahl von Patienten aus unterschiedlichen Gruppen) als Kriterium z.B. für die Wirksamkeit eines Medikamentes.

Wesentlich wichtiger als eine genaue absolute Quantifizierung ist in der Anwendung somit die Wiederholgenauigkeit und insbesondere die Vergleichbarkeit der Messungen, auch wenn unterschiedliche Tomographen verwendet werden.

Diese Grundanforderung bedeutet de facto, eine Standardisierung der Tomographen, insbesondere eine einheitliche räumliche Transferfunktion für alle Tomographen.

Ein derartiger Standard-Tomograph ist aus offensichtlichen Gründen in der Realität nicht verfügbar.

Ausgangspunkt der Erfindung ist nun die Entwicklung eines Verfahrens, das es, ausgehend von einer genauen messtechnischen Charakterisierung eines bestimmten Tomographen anhand einer Bestimmung der jeweiligen Transferfunktion, erlaubt, die von einem Tomographen aufgenommenen Daten dergestalt umzurechnen, als wären sie von einem anderen Tomographen mit bekannter Transferfunktion aufgenommen worden.

Dies erlaubt unter bestimmten Umständen die Definition eines virtuellen Tomographen dergestalt, dass es möglich wird die von unterschiedlichen realen Tomographen aufgenommenen Bilddatensätze jeweils so umzurechnen, als wären sie durch den definierten virtuellen Tomographen aufgenommen worden. Nach dieser Umrechnung werden alle von den unterschiedlichen realen Tomographen aufgenommenen Datensätze direkt quantitativ vergleichbar.

Die hier beanspruchten Verfahren umfassen die Definition eines standardisierten virtuellen Tomographen bzw. eines standardisierten virtuellen bildgebenden Systems mit bekannter und festgelegter Transferfunktion als Grundlage für den Abgleich unterschiedlicher Positronen-Emissions-Tomographen oder anderer medizinisch bildgebender Systeme. Die beanspruchten Verfahren umfassen weiter eine Bestimmung der Abbildungseigenschaften unterschiedlicher Tomographen bzw. bildgebender Systeme ausgehend von geeigneten Referenzmessungen und gegebenenfalls unter Verwendung eines Eichphantoms. Neben besonders günstigen technischen Ausführungen eines derartigen Eichphantoms beschreiben die Ansprüche auch Randbedingungen welche bei den Messungen eingehalten werden sollten, insbesondere mit Blick auf die Linearität der jeweiligen Messungen.

Ausgehend von der Definition des standardisierten virtuellen Tomographen sowie der Bestimmung der Abbildungseigenschaften unterschiedlicher realer Tomographen erlauben die erfindungsgemässen Verfahren dann eine Umrechnung und in der Folge einheitliche und quantitativ vergleichbare Darstellung der von den unterschiedlichen Tomographen oder Systemen aufgenommen Bilddaten so, als wären alle Messungen gleichermassen durch das standardisierte virtuelle System erfolgt.

Das beanspruchte Verfahren zur Handhabung eines virtuellen Tomographen sieht hierfür unter anderem die Verwendung mehrerer mathematisch numerischer Operationen vor, insbesondere eine Kombination aus Konvolution und Dekonvolution, die im Folgenden als **Transkonvolution** bezeichnet wird.

Im Rahmen des beanspruchten Verfahrens wird zunächst der standardisierte virtuelle Tomograph bzw. ein virtuelles bildgebendes System durch die Angabe einer geeigneten räumlichen Transferfunktion definiert und der oder die realen Tomographen bzw. bildgebenden Systeme durch geeignete Messung der jeweiligen räumlichen Transferfunktion (Point Spread Function, *psf* ) charakterisiert.

Der als Transkonvolution bezeichnete numerische Prozess kann dargestellt werden als eine Faltung der jeweils gemessenen Bilder mit der Inversen der Transferfunktion des jeweils aufnehmenden Tomographen (Dekonvolution) gefolgt von einer Faltung des Ergebnisses mit der definierten Transferfunktion des virtuellen Tomographen (Konvolution). Dieser Prozess kann dargestellt werden wie folgt

$$img \otimes psf^{-1} \otimes F^{-1}(H) = img_n \quad ,$$

wobei *img* für das vom jeweiligen Tomographen mit der Transferfunktion *psf* gemessene Bild steht und $F^{-1}(H)$ für die definierte Transferfunktion des standardisierten virtuellen Tomographen. Letztere wird hier als Fouriertransformierte einer Aposidationsfunktion *H* dargestellt.

Das Ergebnis ist jeweils ein standardisiertes Bild $img_n$ das als Ausgangspunkt für normierte bzw. vergleichbare quantitative Bestimmungen dienen kann.

Indem der virtuelle Tomograph bzw. ein virtuelles bildgebendes System als Standard definiert wird, kann das erfindungsgemässe Verfahren damit die quantitative Auswertung von Bilddaten in multi-zentrischen Studien wirksam unterstützen.

Für die Bestimmung der jeweiligen Transferfunktionen der unterschiedlichen Tomographen werden Referenzmessungen unter Verwendung eines dedizierten Festkörper-Phantoms vorgeschlagen.

Für die Handhabung des beanspruchten Verfahrens im Kontext medizinischer bildgebender Systeme, insbesondere Postironen-Emissions-Tomographen, wird bei unverändertem Aufnahmeprozess die Rekonstruktion und gemeinsame Verwaltung von jeweils zwei unterschiedlichen Bilddatensätzen vorgeschlagen. Ein Datensatz wird rekonstruiert für eine optimale Visualisierung der Bilder, ein zweiter Datensatz wird für die quantitativen Messungen möglichst linear rekonstruiert. Dieser zweite Datensatz kann dann ausgehend von der bekannten räumlichen Transferfunktion des jeweiligen Tomographen mit Hilfe einer numerischen Transkonvolution so umgerechnet werden, als wäre die Aufnahme durch das standardisierte virtuelle System erfolgt. Quantitative Messungen ausgehend von dem in dieser Form standardisierte Datensatz sind dann normiert und mit entsprechenden anderen normierten Messungen vergleichbar.

Der hauptsächliche Zweck der Verfahren besteht in der Verbesserung der Aussagefähigkeit klinischer Studien und insbesondere in der Unterstützung und Verbesserung multi zentrischer klinischer Studien.

Neben einer Anwendung im Bereich der Postironen-Emissions-Tomographie kann das Verfahren auch bei anderen komplexen bildgebenden Techniken eingesetzt werden, etwa anderen Tomographen, Ultraschall-Bildgebung oder scannenden Verfahren wie der konfokalen Mikroskopie..

## Relevante Aspekte der Bildgebung und Bildbearbeitung

Ausgehend von den physikalischen Eigenschaften von Licht, insbesondere der Ausbreitung von Licht in Form einer elektromagnetischen Well, sind numerische Methoden im Fourierraum und deren Anwendung in der Bildgebung sehr gut verstanden. Die resultierenden Verfahren wie Wiener Dekonvolution oder iterative Dekonvolution anhand statistischer Kriterien sind in verschiedensten Bereichen der Bildgebung oder Spektroskopie etabliert.

Die Bedeutung von stationären oder nicht stationären räumlichen Transferfunktionen (Point Spread Function, PSF) und physikalischen Grenzen der Auflösung oder der Bildqualität in Folge von stationärem oder nicht stationärem Photonen-Rauschen ist genau definiert. [ERSO07], [GOOD96].

Entsprechend sind die Effekte der diskreten Abtastung in räumlichen, spektralen, oder zeitlichen Koordinaten und der digitalen Repräsentation von Intensitäten in den mathematischen Modellen repräsentiert [BENE01]. Und die Leistungsfähigkeit heutiger Computer erlaubt die routinemässige Verwendung von numerischen Verfahren im Fourierraum auch für grosse Volumendatensätze, insbesondere auch im Bereich der medizinischen Bildgebung.

Trotzdem muss ausdrücklich darauf hingewiesen werden, dass diese numerischen Verfahren, welche ursprünglich im Gebiet der Fourier Optik entwickelt wurden, direkt auf den grundlegenden physikalischen Eigenschaften von Licht im Kontext bildgebende optischer Geräte beruhen. Eine grosse Rolle spielt hier die in den durch eine Poisson Statistik des Photonen-Rauschens gesetzten Grenzen streng lineare Superposition.

Für nicht-optische medizinische bildgebende Geräte und insbesondere für Tomographen sind diese grundlegenden Eigenschaften primär nicht unbedingt erfüllt. Die Anwendung von Fourier-Methoden in der medizinischen Bildgebung kann daher unter Umständen nicht lineare Effekte überhöhen und so eine quantitative Bewertung erschweren.

## Grundlagen der PET und PET/CT Bildgebung

Die PET/CT Bildgebung erfolgt sowohl für den PET- als auch für den CT-Anteil in drei klar getrennten Schritten: Datenerfassung, Berechnung eines Sinogramms und Rekonstruktion der Bilder. Jeder Schritt hat eigene spezielle Eigenschaften und Parameter und trägt zur Leistung des Gesamtsystems bei. Darüber hinaus ist das CT entscheidend für die Dämpfungskorrektur der PET Messungen und geht damit neben dem PET selbst direkt in die Quantifizierung ein. Die technischen Details aktueller klinischer Geräte werden weithin diskutiert [TARA03] [CHER06] [TOWN08].

Die folgenden Abschnitte fokussieren auf die Linearität der genannten Schritte als Grundlage quantitativer Messungen.

### PET Datenerfassung

Klinische PET/CT Systeme profitieren weiter von der fortschreitenden technischen Entwicklung. Bessere und schnellere Szintillatoren, Photodetektoren, Elektronik und Computer erlaubten den Übergang von 2D Erfassung der Koinzidenz-Signale zu einer vollen 3D Erfassung und jetzt weiter zur "time-of-flight" (TOF) Erfassung. Trotzdem hat sich das grundsätzliche Prinzip der Koinzidenzmessung nicht verändert und innerhalb der durch das Photonenrauschen und das Hintergrundrauschen gesetzten Grenzen bleibt der Messprozess linear. Obwohl einige Parameter des Messprozesses über das Gesichtsfeld bzw. Messvolumen variieren sind die genauen Eigenschaften der Datenerfassung insgesamt ausgehend von einer festen Anordnung und stabilen Paramtern der Szintillatoren und Detektoren sehr gut definiert. Daher und basierend auf dem Messprinzip kann davon ausgegangen werden, dass die PET Datenerfassung innerhalb weiter Grenzen linear ist.

### Berechnung des PET Sinogramms

Jedes während der Datenerfassung registrierte Koinzidenzereignis wird als eine Intensitätsverteilung entlang einer "line of response" (LOR) bzw. einer "tube of response" (TOR) interpretiert, welche die beteiligten Szintillatoren verbindet.

Jede LOR bzw. TOR kann numerisch durch einen Winkel sowie einen Versatz gegenüber dem Zentrum des Gesichtsfeldes dargestellt werden. Ausgehend von Werten für Winkel und Versatz als Koordinaten wird das Sinogramm dann durch numerische Projektion der Koinzidenzereignisse in ein 2- oder 3-dimensinales Koordinatensystem i.e. ein entsprechendes Datenvolumen erzeugt.

Der Prozess kann gleichzeitig verwendet werden um eine Dämpfungskorrektur vorzunehmen indem die relative Gewichtung von LORs entsprechend einer vorher berechneten Dämpfungsmaske erhöht wird um die Dämpfung zu kompensieren. Weiter können über das TOR Konzept gewisse Korrekturen einfliessen indem numerisch der Durchmesser der TOR variiert wird, um z.B. Eigenheiten der geometrischen Anordnung der Detektoren oder unterschiedlichen Nachweisempfindlichkeiten einzubeziehen [HERR07]. In ähnlicher Weise kann time of flight information (TOF) eingebunden werden um den relativen Beitrag eines Ereignisses auf nur einen Teilbereich entlang der LOR oder TOR einzugrenzen. In jedem Fall muss während oder nach der Projektion eine Interpolation und resampling erfolgen so, dass der resultierende Datensatz unabhängig von der exakten geometrischen Anordnung der Detektoren in einer für die folgenden numerischen Verfahren geeigneten Form bereit gestellt werden kann. Ein geeignetes und häufig verwendetes Verfahren ist das "Fourier rebinning" (FORE)

Die Erstellung des Sinogramms einschliesslich sowohl der Dämpfungskorrektur als auch der Berücksichtigung der time of flight Information und der geometrischen Eigenheiten der jeweiligen Szintillatoren sowie das Fourier-Rebinning könneninnerhalb der durch Rauschen gesetzten Grenzen als lineare Prozesse betrachtet werden. Die verschiedenen Korrekturen können jedoch zu Abweichungen sowohl von der zu erwartenden Poisson-Statistik des Rauschens führen als auch zu einer bezogen auf das Gesichtsfeld des Tomographen inhomogenen Verteilung des Rauschens.

Abgesehen von den genannten Effekten auf die Eigenschaften des Rauschanteils bleibt es möglich für dieses "primäre" Sinogramm eine zwar nicht stationäre wohl aber lineare System Transfer Funktion zu definieren. Auf Basis einer derartigen Transferfunktion sind somit quantitative Messungen möglich.

In einem weiteren Schritt können numerische Methoden ("sinogram restoration methods") etwa zur Erhöhung der Auflösung des Sinogramms durch inverse Filterung angewendet werden. Die Anwendung etwa eines Wiener Filters (Wiener Dekonvolution) kann sich positiv auf die räumliche Auflösung auswirken erhöht aber im Gegenzug den Rauschanteil und führt zu einer weiteren Verzerrung der statistischen Eigenschaften des Rauschanteils [HERR06].

Fortgeschrittenere auf das Sinogramm angewandte numerische Methoden, etwa eine iterative Dekonvolution ("maximum likelihood deconvolution") ausgehend von einer nicht stationären Transferfunktion, können die Auflösung des Sinogramms weiter verbessern sind aber unter anderem abhängig vom jeweils lokalen Rauschanteil und den vorliegenden Daten selbst. Diese Methoden können somit starke nicht lineare Effekte haben und sind daher im Rahmen quantitativer Messungen zu vermeiden.

## *PET Bild Rekonstruktion*

Ausgehend vom Sinogramm wird im dritten Schritt das eigentliche Bild, bzw. das aus der Messung resultierende Datenvolumen berechnet (Rekonstruktion). Die gefilterte Rückprojektion ("Filtered back projection", FBP) ist ein hierfür geeignetes einfaches, zuverlässiges, schnelles und vor allem lineares Verfahren. Bei der Bildgebung erreicht eine Rekonstruktion durch FBQ eine annehmbare räumliche Auflösung bezogen auf die Halbwertsbreite ("full width half maximum, FWHM) der resultierenden PSF. Allerdings zeigt die PSF einen relativ breiten Fuss welcher eine Bildunschärfe bewirkt und quantitative Messungen behindert. Dies insbesondere, da der Fuss der PSF sich in Volumendatensätzen in allen 3 Dimensionen ausbreitet und einen entsprechenden Überlaufeffekt zwischen Voxeln bewirkt ("spill in", "spill out").

Im Kontext quantitativer Messungen ist zu betonen, dass FBP grundsätzlich eine lineare Operation ist und ausgehend von einem geeigneten Sinogramm zu einer stabilen und reproduzierbaren PSF führt.

Soweit nicht bereits im Sinogramm geschehen kann eine Dämpfungskorrektur auch mit der FBP verknüpft werden oder auf das resultierende Bild angewendet werden.

Moderne, iterative, Methoden der Rekonstruktion nach statistischen Kriterien wie etwa "maximum likelihood expectation maximum" (MLEM) oder "ordered subset expectation maximum" (OSEM) sind zum Standard für die Rekonstruktion von PET Bildern avanciert. Diese Methoden implementieren im Prinzip zunächst eine Methode welche umgekehrt die Berechnung eines Sinogramms ausgehend von einem Bild und den bekannten Eigenschaften des jeweiligen Tomographen erlaubt. Das Verfahren verändert dann iterativ ein zunächst grobes Bild solange, bis das zum jeweiligen Bild berechnete Sinogramm eine nach statistischen Kriterien möglichst gute Übereinstimmung mit aus der dem tatsächlichen Messung erzeugten Sinogramm zeigt. Durch Angabe geeigneter Randbedingungen wir der Prozess so gesteuert, dass ein geeigneter Kompromiss zwischen erhöhter räumlicher Auflösung, Unterdrückung des Fusses der Transferfunktion und dem resultierendem Bildrauschen erreicht wird. In Folge der statistischen Natur der iterativen Mehoden reagieren diese jedoch empfindlich auf Variationen im Rauschanteil der ursprünglichen Messung sowie auf Eigenschaften der Bildinformation selbst.

Während die iterativen Methoden daher eine im Vergleich zur FBP höhere räumliche Auflösung zeigen und damit auch den störenden Einfluss des PVE bei  quantitativen Messungen an verhältnismassig kleinen Objekten verringern können, bleibt jeweils unklar wie weit diese Verbesserungen tatsächlich reichen.

Mit zwar insgesamt verbesserter Bildgebung und Messgenauigkeit aber tatsächlich im jeweiligen Einzelfall unbekannter und variabler PSF bleibt der Nutzen der iterativen Methoden bezüglich der erforderlichen Vergleichbarkeit von Messungen insbesondere im Kontext multi zentrischer Studien beschränkt.

**Der Standardized Uptake Value (SUV)**

In Kontext der PET beschreibt das Konzept des SUV die Akkumulation von radioaktiven Markern im Gewebe abhängig von der injizierten Gesamtdosis und dem Gewicht des Patienten. Der SUV ist als Messgröße weit verbreitet und soll vergleichbare Resultate liefern. Allerdings zeigt das Konzept seit seiner Einführung eine bemerkenswerte Entwicklung und Variabilität. Die frühen Definitionen beschreiben gemessenen Aktivität pro Gramm Gewebe und Gewicht des Patienten bezogen auf injizierte Aktivität [OLDE74][WOOD75]. Die ursprüngliche Definition des SUV [STRA91] war definiert als Konzentration im Gewebe (mCi/g) pro injizierter Dosis (mCi/g) pro Körpergewicht des Patienten (g). Es resultiert also ein dimensionsloser absoluter Wert welcher als Verhältnis zwischen der in einem definierten Volumen gemessenen Aktivität zu einer gemittelten Aktivität bezogen auf den ganzen Körper betrachtet werden kann. Eine aktuellere Definition im klinischen Kontext kann umschrieben werden wir folgt:

"Der SUV ist eine gemessene Aktivitätskonzentration in Bq/ml innerhalb eines definierten Volumens ("Volume of interest") bezogen auf die injizierte Aktivität in Bq pro Körpergewicht des Patienten in g gemessen zu einem definierten Zeitpunkt nach der Injektion."

Diese Definition führt nicht auf einen dimensionslosen Wert sondern resultiert formal in einer Einheit g/ml.

Andere Varianten verwenden die Körperoberfläche des Patienten als Bezugsmaß für die Normierung [THIE04].

Die vorwiegend verwendete Formel für die Berechnung des SUV ausgehend von "activity concentration" innerhalb eines "volume of interest" (ACvoi), der injizierten Dosis (FDGdose) und dem Körpergewicht (BW) wird von Boellard [BOEL09] angegeben als:

$$SUV = \frac{ACvoi\,[kBq/ml]}{FDGdose\,[MBq]/BW\,[kg]} \qquad\qquad (1)$$

Leider schlagen die Definitionen keine Methode für die Beschreibung des "Volume of Interest" (VOI) vor, was sehr unterschiedliche Varianten der Durchführung der tatsächlichen Messung mit entsprechend unterschiedlichen Ergebnissen erlaubt [BOEL03]. Der äußert kritische Einfluss der Definition des zu messenden Volumens "Volume of Interest") auf das Ergebnis der Messung ist gut bekannt [KEYE95].

Daher wird als mit Abstand häufigste Methode der SUV Messung die Bestimmung des $SUV_{max}$ verwendet, i.e. es wird als Maß für die Aktivität der größte Wert, den ein einzelnes Voxel innerhalb des VOI zeigt verwendet. Durch die einfache Übernahme dieses Maximalwertes wird die Notwendigkeit einer genaueren Definition des eigentlichen VOI umgangen und damit der Einfluss etwa einer manuellen Definition des VOI oder eine Umschreibung des VOI anhand eines Schwellenwertes vermieden [BOEL09]. Dies trotz der öffensichtlichen Nachteile, die bei einer auf einem einzelnen Voxel basierenden Messung auftreten.

**Messung von $SUV_{max}$**

Die Aktivitätskonzentration ist statistisch definiert als die mittlere Anzahl radioaktiver Zerfälle pro Zeit und Volumen. Die tatsächliche Messung einer Aktivitätskonzentration ist daher zwingend an eine geeignete Definition des jeweiligen Messvolumens gebunden. Streng genommen ist es daher gar nicht möglich eine Aktivitätskonzentration anzugeben ohne gleichzeitig das entsprechende Messvolumen zu beschreiben. Weiter impliziert die Definition, dass die Messung einer Poisson Statistik unterliegt, i.e. die Messgenauigkeit jedenfalls durch Photonen Rauschen ("shot noise") begrenzt ist bzw. entsprechend durch die Anzahl erfasster Zerfälle innerhalb des Messvolumens während der Messzeit.

Die Anzahl der erfassten Zerfälle kann dabei als ein der Nachweisempfindlichkeit entsprechender Anteil der tatsächlichen Zerfälle betrachtet werden vermindert entsprechend der jeweiligen Absorption.

Abweichend von den in der Literatur beschriebenen vereinfachenden Modellen muss daher die Definition der Aktivitätskonzentration jeweils eine Beschreibung des Messprozesses enthalten und damit direkt Bezug nehmen auf die Parameter des jeweils verwendeten PET/CT Systems. Bezogen auf die Messung anhand eines einzelnen Voxels muss eine Definition dann etwa wie folgt lauten:

*"Die gemessene Aktivitätskonzentration bezogen auf ein einzelnes Voxel eines PET-Bilddatensatzes repräsentiert die Anzahl der detektierten radioaktiven Zerfälle über ein entsprechend der effektiven räumlichen Transfer Funktion des Tomographen gewichtetes Mittel einer Region zentriert um das jeweilige Voxel und korrigiert bezüglich der Nachweisempfindlichkeit des Systems und bezüglich der jeweils von der Messsituation abhängigen Dämpfung. "*

Somit ist eine Messung des $SUV_{max}$ zunächst untrennbar verbunden mit der räumlichen Transferfunktion (PSF) des Tomographen, welche das Messvolumen definiert, und weiter zumindest bezüglich der statistischen Genauigkeit der Messung abhängig von der Empfindlichkeit des Tomographen und der jeweiligen Dämpfung.

Bei räumlichen Variationen der Aktivitätskonzentration in der Größenordnung der Voxelgrösse wird das Messergebnis zusätzlich durch den Samplinprozess gestört, da die jeweiligen räumlichen Maxima einer Aktivitätskonzentration nicht mit den jeweiligen Zentren der Voxel selbst zusammenfallen sondern bezogen auf ihre Position innerhalb des Voxelrasters eine zufällige Verteilung haben.

**Partial Volumen Effekt**

Der partial Volumen Effekt (PVE) ("partial volume effect" oder "spill in" und "spill out") ist der wichtigste Faktor, welcher quantitative PET/CT Messungen limitiert [SORE07]. Der PVE wird verursacht durch die begrenzte räumliche Auflösung des Tomographen und in der Regel als abhängig von der Halbwertsbreite ("Full Width Half Maximum") (FWHM) der PSF beschrieben. In der Praxis betrifft der PVE alle Messungen an Strukturen mit Durchmessern kleiner als etwa dem 3-fachen der FWHM. Entsprechend werden Messungen ganz allgemein unzuverlässig, wenn die Aktivitätsverteilung Strukturen mit Raumfrequenzen unter etwa dem 3-fachen der FWHM zeigt.

Ursache des PVE ist, dass - wie oben beschrieben - die Messung der Aktivitätskonzentration immer ein gewichtetes Mittel über eine Region darstellt, welche durch die PSF beschrieben wird. Die Ausläufer der PSF reichen jedoch weit über die FWHM hinaus. Bei 3-dimensionaler Bildgebung wird der Effekt noch dadurch verschärft, dass die Ausläufer der PSF in allen 3 Dimensionen in das umgebende Volumen reichen und daher mit dem Quadrat des Abstandes zum Zentrum des jeweiligen Voxels höher gewichtet werden. Der Durchmesser der Region, welche wesentlich zum Signal beiträgt ist somit deutlich grösser als die FWHM der PSF. Dieser Einfluss der Umgebung auf jede lokale Messung verfälscht entsprechend die Messergebnisse insbesondere bei Messungen der Aktivitätskonzentration an kleinen Strukturen. Weiter wird der Einfluss der Umgebung auf die jeweilige Messung durch die FWHM nicht hinreichend beschrieben. Vielmehr ist auch eine Kenntnis der genauen Form der PSF und insbesondere auch der Randbereiche der PSF erforderlich. Eine genaue Kenntnis der PSF erlaubt in gewissen Grenzen eine Korrektur des PVE durch Dekonvolution. Insbesondere ist der PVE im Prinzip vollständig durch die jeweilige effektive PSF der Messung vollständig. definiert.

**Klinische PET Protokolle für quantitative Messungen**

PET Aufnahmen für eine quantitative Analyse erfordern die strikte Einhaltung von .Untersuchungsprotokollen, welche die Vorbereitung der Patienten, die Präparation und Injektion des radioaktiven Markers, die technischen Parameter der Aufnahme selbst, die Verfahren zur Rekonstruktion der Bilder sowie natürlich die Methoden der numerischen Analyse der Daten zur Bestimmung des jeweiligen Messwertes festlegen. Weiter ist eine Eichung bzw. ein Abgleich der verwendeten Tomographen erforderlich.

Insbesondere im Kontext multi zentrischer medizinischer Studien werden derzeit die Anforderungen an einheitliche Untersuchungsprotokolle, an den Abgleich der Tomographen und an die Methoden zur Bestimmung quantitativer Werte intensiv diskutiert [WAHL09], [BOEL09], [BOEL09b], [DELB06], [SHYA08], [SCHE09]. Obwohl diese Vorschläge bereits umfangreiche Vorgaben für die erforderlichen Untersuchungsprotokolle beinhalten, unterliegen die Messwerte infolge der jeweils unterschiedlichen Abbildungseigenschaften der in den jeweiligen Institutionen verfügbaren PET/CT Systeme dennoch grossen Variationen [WEST2006].

Das Problem wird dadurch verschärft, dass die System über eine wachsende Anzahl variabler technischer Parameter verfügen, welche eine Optimierung der Bildgebung für bestimmte Anwendungen zulassen, gleichzeitig aber eine quantitative Auswertung der so manipulierten Bilder behindern.

Um diese Situation zu verbessern zielen die hier beanspruchten Verfahren auf eine Kompensation der Unterschiede in den Abbildungseigenschaften der jeweiligen bildgebenden Systeme.

Es wird vorgeschlagen im Kontext quantitativer Messungen zukünftig für jede PET Aufnahme zwei Rekonstruktionen auszuführen und die entstehenden Bildpaare parallel weiter handzuhaben. Eine erste Rekonstruktion wird für eine optimale Bildgebung unter Nutzung der iterativen numerischen Verfahren zur Erhöhung von räumlicher Auflösung und Kontrast durchgeführt, entsprechend der klinischen Routine. Für die quantitativen Messungen wird parallel eine zweite Rekonstruktion durchgeführt, welche sich auf die weitgehend linearen Verfahren beschränkt. Diese zweite Rekonstruktion - im folgenden als lineare Rekonstruktion bezeichnet - ist als Ausgangspunkt für quantitative Messungen unter Verwendung der hier beanspruchten Verfahren geeignet, da die Beschränkung auf eine lineare Rekonstruktion eine stabile Transferfunktion des Systems gewährleisten kann.

**Abgleich (cross calibration) unter Verwendung von "Recovery Factors".**

Die absolute Eichung von PET Systemen bezogen auf die Messung einer Aktivitätskonzentration ist eine anspruchsvolle Aufgabe [GEWO02].

Trotzdem ist ein gegenseitiger Abgleich unterschiedlicher PET Systeme bezogen auf grosse homogene Quellen wie etwa ein käuflich erhältliches mit 68Ge dotiertes Zylinder Phantom (20 cm Durchmesser) relativ einfach durchführbar [SCHE09].

Dies ist tatsächlich möglich, weil der Durchmesser des homogenen Phantoms den Durchmesser der PSF der PET Systeme bei weitem übersteigt.

Bei kleineren Objekten funktioniert diese Art des Abgleichs nicht mehr da die Messungen durch den PVE beeinflusst werden, welcher sowohl in Abhängigkeit von der FWHM der PSF als auch in Abhängigkeit von der genauen Form der PSF erheblich variiert. Da die PSFs unterschiedlicher Tomographen sich deutlich voneinander unterscheiden und der PVE nicht ohne zusätzliche Information korrigiert werden kann, ist ein einfacher Abgleich unterschiedlicher PET Systeme nicht möglich.

Ein aktueller Ansatz, diese Problematik zu überwinden, ist die Messung von "Recovery Factors" anhand von Phantom-Messungen an Kugeln mit homogener Aktivitätskonzentration aber unterschiedlicher Grösse.

Für den jeweiligen Tomographen wird jeweils bestimmt, welcher Anteil (="Recovery Factor") der tatsächlichen Aktivitätskonzentration im Zentrum der jeweiligen Kugel noch gemessen wird. In anderen Worten es wird der PVE für homogene Kugeln bekannter Grösse in homogener Umgebung durch eine Messung bestimmt.

Bei späteren Messungen kann dann unter der Annahme, dass das zu messende Objekt in etwa kugelförmig ist, eine homogene Aktivitätskonzentration in homogener Umgebung darstellt und der Durchmesser anderweitig - etwa im CT Bild -bestimmt werden kann, mit Hilfe des jeweils passenden Recovery Factors auf die tatsächliche Aktivitätskonzentration zurückgeschlossen werden [SHYA08]. Diese Methode unterliegt natürlich der Einschränkung, dass die Ergebnisse nur bei kugelförmigen, homogenen Objekten genau sind, weiter der Einschränkung, dass die genaue Grösse der Kugel anderweitig bestimmt werden muss. Weiter treten evtl. Ungenauigkeiten bei unterschiedlichen Tomographen mit unterschiedlichen PSF in ganz unterschiedlicher Weise auf, was einen Vergleich der Ergebnisse für unterschiedlicher Tomographen stark erschwert.

Die hier beanspruchten Verfahren zielen daher primär nicht auf eine unbestimmte absolute Korrektur des PVE sondern direkt auf einen Vergleich der Messergebnisse unterschiedlicher Tomographen.

Grundlage der beanspruchten Verfahren ist daher nicht die Bestimmung von Recovery Faktoren sondern -ggf. unter Verwendung eines geeigneten Phantomsdie Bestimmung der tatsächlichen PSF der unterschiedlichen bildgebenden Systeme.

**Festkörper Phantom (Solid State Phantom)**

Kalibrationsmessungen unter Verwendung eines Phantoms oder anderer Strahlenquellen sind eine Grundlage der Qualitätssicherung für PET Systeme [DAUB02]. In der Regel werden unter Verwendung von unterschiedlich grossen Kugeln mit homogener Aktivitätskonzentration entsprechende "Recovery Factors" bestimmt, wie oben beschrieben. Obwohl gelegentlich zusätzliche Messungen mit Punktquellen vorgenommen werden um die FWHM zu messen, wird die jeweilige Form der PSF in der Regel nicht genauer gemessen oder bestimmt.

Zur Bestimmung der PSF wird hier, abweichend von der ansonsten nahe liegenden Verwendung einer Punktquelle, die Verwendung eines neuartigen Festkörper Phantoms vorgeschlagen. Dieses Phantom unterscheidet sich von den derzeit verfügbaren Standardphantomen mit aktiven Kugeln etwa gemäss der NEMA/IEC 2001 Norm in drei wesentlichen Punkten:

- Es handelt sich um ein Festkörperphantom, das leicht transportabel ist und dessen Kugeln nicht von Messung zu Messung neu befüllt werden müssen.
- Das Phantom enthält verglichen mit dem Standardphantom mehr, welche zusammen einen grösseren Bereich an Durchmessern abdecken.
- Und die Kugeln sind auch einzeln in ganz unterschiedlichen Anordnungen handhabbar, was eine schnelle Realisierung von an die jeweilige Anwendung angepassten speziellen Phantomen erlaubt, etwa eine Positionierung in der Nähe von Absorbern oder in Hohlräumen oder beides.

Die Verwendung eines Festkörperphantoms auf Basis des relativ langlebigen 68Ge Isotops vermeidet das ansonsten erforderliche wiederholte Befüllen der Kugeln und vereinfacht die Handhabung der Kugeln deutlich. Damit wird eine ganze Reihe von Fehlermöglichkeiten vermieden und insbesondere auch die Strahlenbelastung der mit der Handhabung des Phantoms betrauten Personen deutlich vermindert.

Weiter ist gegenüber einem mit Aktivität in flüssiger Form zu befüllenden Phantom die Handhabung des Festkörperphantoms insgesamt deutlich einfacher und dies unabhängig von der Anzahl der Kugeln, die es enthält. Davon ausgehend und um eine genauere Bestimmung der PSF zu ermöglichen und unter Berücksichtigung der höheren räumlichen Auflösung moderner PET/CT Systeme wurde die Reihe der Kugeln hin zu kleineren Durchmessern erweitert.

Das für die Evaluation der beanspruchten Verfahren hergestellte Phantom verwendet 12 Hohlkugeln welche Volumen von 16ml bis herunter zu 1/128 ml umfassen entsprechend Durchmessern von rund 31 mm bis herunter zu 2,5 mm. Die Kugeln wurden homogen befüllt mit einem Epoxid-Harz versetzt mit einer Aktivitätskonzentration von 0,1 MBq/ml. Nach dem Aushärten des Harzes und zusätzlicher Versiegelung der Öffnungen zum Befüllen der Kugeln, können diese ohne Gefahr einer Kontamination in einfacher Weise gehandhabt werden.

Die Kugeln wurden innerhalb eines Plexiglass Zylinders von 22 cm Durchmesser Durchmesser auf 2 konzentrischen Kreisen montiert. Auf dem äusseren Kreis mit rund 14 cm Durchmesser sind die 6 grösseren Kugeln montiert, auf dem inneren Kreis mit rund 7 cm Durchmesser die 6 kleineren Kugeln. Der Plexiglass-Zylinder kann mit Wasser befüllt werden.

Weiter sind die einzelnen Kugeln so gestaltet und mit einem geeigneten Gewinde zur Befestigung versehen, dass sie in einfachster Weise innerhalb anderer Anordnungen verwendet werden können. Insbesondere ist es in einfacher Weise möglich im Kontext konkreter medizinischer Fragestellungen eine Anordnung aufzubauen welche bzgl. der radiologischen Eigenschaften bestimmte anatomische Gegebenheiten simuliert. Im Kontext etwa medizinischer Studien unter Verwendung von PET/CT Aufnahmen, kann mit derartigen speziell an die Fragestellung angepassten Phantomen die Genauigkeit des Abgleichs unterschiedlicher Geräte für eben diese Fragestellung weiter erhöht werden.

**Bestimmung der räumlichen Transferfunktion (PSF)**

Der Prozess der Bildgebung wird üblicherweise so dargestellt, dass das Bild (img) als Konvolution (Faltung) eines das Objekt repräsentierenden Urbildes (obj) mit einer für das abbildende Gerät spezifischen räumlichen Transferfunktion (point spread function, PSF).

$$obj \otimes psf = img \qquad\qquad (2)$$

Offensichtlich kann die PSF (psf) daher als das Bild einer Punktquelle (point) im Zentrum des Koordinatensystems definiert werden.

$$point \otimes psf = psf \qquad\qquad (3)$$

Üblicherweise wird die PSF eines bildgebenden Systems bzw. eines Tomographen ausgehend von Gleichung (3) anhand des Bildes einer Punktquelle gemessen, i.e. unter Verwendung einer Quelle deren Durchmesser sehr viel kleiner ist als die FWHM der zu vermessenden PSF.

Tatsächlich ist diese Methode jedoch nicht immer optimal, da die Abbildung einer Punktquelle im Allgemeinen nicht der tatsächlichen Anwendung des Systems entspricht. Typischerweise werden Objekte mit Strukturen endlicher Grösse abgebildet und es erscheint plausibel, die PSF auch anhand von Objekten zu bestimmen deren Grösse im Bereich der Grössen der üblicherweise abzubildenden bzw. interessierenden Objekte liegt.

Für die Bestimmung der PSF eines PET Systems wird daher eine Messung am oben beschriebenen Phantom i.e. Messungen an homogenen Kugeln bekannter Grösse vorgeschlagen ausgehend direkt von Gleichung (2).

Für den Fall eines genau bekannten Objektes und damit eines genau bekannten Urbildes (obj) und bei vorliegendem, gemessenem Bild (img) kann die PSF durch numerische Dekonvolution i.e. durch Faltung des Bildes (img) mit der invers Fourier-Transformierten des Objektes ($obj^{-1}$) bestimmt werden gemäss fer folgenden Gleichung:

$$psf = img \otimes obj^{-1} \qquad\qquad (4)$$

In der Praxis ist eine numerische Bestimmung der PSF durch iterative numerische Verfahren der Dekonvolution ausgehend von Gleichung 2 ohne weiteres möglich. Diese Methode ist offensichtlich robuster als die sonst übliche Methode unter Verwendung einer Punktquelle, weil eine grössere Anzahl an Voxeln zum Ergebnis beiträgt und weil die Ausgangsmessungen an mehreren unterschiedlich grossen Kugeln dem Kontext der tatsächlich angestrebten klinischen Messungen nahe kommen.

Ausgehend von einer einzelnen Messung des beschriebenen Phantoms kann daher sowohl die effektive PSF als auch ein absolutes Mass für den Abgleich unterschiedlicher PET-Systeme gewonnen werden. Weiter resultiert ein Mass für die Empfindlichkeit etwa in Form eines bestimmbaren Signal zu Rausch Verhältnisses bezogen auf Messsituationen welche durch das Phantom repräsentiert werden können.

Anzumerken ist, dass die PSF eines PET-Systems in der Regel nicht stationär ist und mit wachsendem transaxialem Abstand vom Zentrum des Sichtfeldes einer systematischen Verbreiterung unterliegt. Je nach Anforderungen muss diese räumliche Variation der PSF entsprechend berücksichtigt werden. Dies kann geschehen anhand mehrerer Messungen des Phantoms an unterschiedlichen Positionen im Sichtfeld des Tomographen und die Berechnung und Darstellung einer ortsabhängigen PSF durch geeignete Interpolation bzw. Extrpolation der jeweils an den unterschiedlichen Orten aus der Messung bestimmten lokalen PSFs oder durch eine mathematische Transformation der gemessenen lokalen PSF entsprechend bekannter technischer Eigenschaften des jeweiligen Tomographen.

**Transkonvolution**

Wie oben dargestellt ist die Messung des SUV untrennbar mit Eigenschaften des Tomographen verknüpft. Insbesonders steht die Bestimmung des $SUV_{max}$ in direktem Zusammenhang mit der PSF des jeweiligen Tomographen. Streng genommen ist eine absolute Bestimmung des SUV zumindest für kleine Objekte nicht nur unmöglich sondern gar nicht erst definierbar, da die Messung einer Aktivitätskonzentration immer ein gewisses Messvolumen voraussetzt. Wie beschrieben wird dieses Messvolumen durch die PSF des jeweiligen Tomographen definiert.

Um bei SUV Messungen valide und vergleichbare Werte zu erhalten wäre also eine Standardisierung der PSF der verwendeten Tomographen erforderlich, bzw. eben die Verwendung eines Standard-Tomographen für alle Messungen. Leider haben die konkurrierenden Hersteller von PET/CT Systemen aus verständlichen Gründen kein Interesse an der Herstellung standardisierter Tomographen mit für alle Hersteller jeweils identischen Eigenschaften.

Zur Überwindung dieser Problematik wird ein neues numerisches Verfahren vorgeschlagen, dass im folgenden als Transkonvolution bezeichnet wird.

Zur Erklärung des Verfahrens werden zunächst zwei unterschiedliche bildgebende Systeme definiert wie folgt:

$$obj \otimes psf_1 = img_1 \qquad\qquad (5a)$$

$$obj \otimes psf_2 = img_2 \qquad\qquad (5b)$$

Die zwei unterschiedlichen räumlichen Transfer Funktionen $psf_1$ und $psf_2$ repräsentieren die beiden unterschiedlichen Tomographen bzw. unterschiedlicher bildgebender Systeme, welche entsprechend die zwei unterschiedliche Bilder $img_1$ und $img_2$ des selben Objektes $obj$ erzeugen.

Mathematisch theoretisch könnte das Objekt bei bekannter PSF durch Dekonvolution, d.h. eine inverse Filterung folgender Form rekonstruiert werden:

$$img_1 \otimes psf_1^{-1} = obj \qquad\qquad (6)$$

Für reale Messungen mit limitierter Anzahl Stützpunkte (Sampling), limitierter Auflösung und in Anwesenheit von Rauschen ist eine derartige Dekonvolution nur sehr begrenzt möglich. Insbesondere kann die Inverse der Transferfunktion ($psf_1^{-1}$) in der Regel nicht numerisch dargestellt werden.

Aus den Gleichungen (5) und (6) folgt jedoch:

$$img_1 \otimes psf_1^{-1} \otimes psf_2 = img_2 \qquad\qquad (7)$$

Diese Formel stellt dar, wie bei bekannten räumlichen Transfer Funktionen der jeweiligen bildgebenden Systeme (psf1, psf2) das Bild eines Objektes, wie es von einem ersten bildgebenden System erstellt wurde ( img1 ), so transformiert werden kann, dass ein zweites Bild entsteht ( img2 ), das dem Bild des Objektes entspricht, wie es von einem zweiten unterschiedlichen bildgebenden System erstellt worden wäre.

Zur weiteren Erklärung wird die Transkonvolution-Funktion ( tf )eingeführt wie folgt:

$$tf \equiv psf_1^{-1} \otimes psf_2 \qquad\qquad (8)$$

$$img_1 \otimes tf = img_2 \qquad\qquad (9)$$

Im Allgemeinen kann die Inverse einer PSF numerisch nicht dargestellt werden, so dass ( $psf_1^{-1}$ ) nicht vollständig definiert werden kann. Die durch die Gleichungen (8) und (9) beschriebenen Faltungen können jedoch nach Fourier-Tarnsformation im Frequenzraum in einfacher Weise dargestellt werden wie folgt:

$$F(tf) \equiv F(psf_1^{-1}) \cdot F(psf_2) \qquad\qquad (10)$$

$$F(img_1) \cdot F(tf) = F(img_2) \qquad\qquad (11)$$

Ausgehend von einer räumlichen Transfer-Funktion *psf* ist der Term |*F(psf)*| definiert als die entsprechend Modulations-Transfer-Funktion (MTF) des Systems bezogen auf die übertragenen Raumfrequenzen. Für jedes realistische bildgebende System wird die MTF zu höheren Raumfrequenzen hin abfallen und schließlich oberhalb einer bestimmten Grenzfrequenz unter einen jeweils vorhandenen Rauschpegel fallen. Die Inverse einer räumlichen Transferfunktion kann offensichtlich nur für Werte unterhalb dieser Grenzfrequenz sinnvoll bestimmt werden. Diese Tatsache ist der tiefere Grund für die prinzipiellen Grenzen der Dekonvolution.
Der mathematische und messtechnische Hintergrund wurde im Detail z.B. im Kontext der inversen Filterung insbesondere des Wiener-Filters ausgearbeitet.
Im Bereich der numerischen Bildbearbeitung wird das Verfahren der Faltung (Konvolution) unter Verwendung der Fourier-Transformation häufig für die Filterung von Bildern genutzt. Ein derartiges Filter repräsentiert eine räumliche Transfer Funktion (psf) im Frequenzraum durch eine "Window-Funktion", welche tatsächlich eine entsprechende MTF repräsentiert. Wichtig ist dabei, dass als Window-Funktionen sinnvollerweise Apodisations-Funktionen Verwendung finden können, welche oberhalb einer bestimmten Grenzfrequenz auf 0 abfallen.
Eine der häufig verwendeten Window-Funktionen ist die Hanning Funktion *H* ("raised cosine Window") welche eingesetzt als Filter durch vollständige Unterdrückung von Raumfrequenzen oberhalb einer bestimmten Grenzfrequenz eine Glättung bewirkt aber gleichzeitig durch ihre gleichmässige Form im gefilterten Bild verhältnismässig geringe Artefakte wie etwa Überschwinger an Kanten verursacht.
Eine Hanning Funktion mit variabler Breite und einer frei bestimmbaren Grenzfrequenz $\omega$ kann definiert werden wie folgt:

$$H_\omega(v) \equiv 0.5 + 0.5 \cdot \cos(\pi v / \omega) \quad \text{für} \quad |v| \le \pi \qquad\qquad (12a)$$

$$H_\omega(v) \equiv 0 \quad \text{für} \quad |v| > \pi \qquad\qquad (12b)$$

Eine derartige Hanning Funktion oder eine vergleichbare Apodisations-Funktion ist geeignet für eine Verwendung als MTF eines virtuellen bildgebenden Systems. Entsprechend kann als Beispiel unter Verwendung einer Hanning-Funktion $H_\omega$ eine normalisierte Transkonvolution definiert werden wie folgt:

$$F(img) \cdot F(psf^{-1}) \cdot H_\omega = F(img_n) \qquad\qquad (13)$$

$$F(ntf_\omega) \equiv F(psf^{-1}) \cdot H_\omega \qquad\qquad (14)$$

$$img \otimes psf^{-1} \otimes F^{-1}(H_\omega) = img_n \qquad\qquad (15)$$

Dabei ist *img* das Bild, welches durch das jeweilige bildgebende System mit der räumlichen Trannsfer Funktion *psf* aufgenommen wurde. Die Funktion $ntf_\omega$ ist die für das jeweilige System ermittelte normalisierte Transkonvolutions Funktion ausgehend von der jeweiligen räumlichen Transfer Funktion und $img_n$ ist das resultierend normalisierte Bild, wie es von einem standardisierten virtuellen Tomographen aufgenommen worden wäre. Insofern der Parameter $\omega$ nicht zu gross gewählt wird, das heißt innerhalb der Grenzen welche durch die räumlichen Grenzfrequenzen der beteiligten bildgebenden Systeme gesetzt werden, ist eine numerische Berechnung in jedem Fall möglich.

**Interpolation nicht stationärer räumlicher Transferfunktionen**

Im Falle von Tomographen oder anderen komplexen bildgebenden Systemen ist die räumliche Transferfunktion nicht stationär i.e. ortsabhängig. Die Berechnung der Faltungen wird dadurch komplizierter, kann aber numerisch ohne weiteres entsprechend den angegebenen Verfahren durchgeführt werden. Gegebenenfalls sind die Verfahren durch geeignete Interpolation oder gegebenenfalls Stückweise Berechnung an die räumlich variierenden räumlichen Transferfunktionen zu adaptieren.

**Anwendung des virtuellen Tomographen in multi-zentrischen PET Studien**

Nach Festlegung eines geeigneten Untersuchungsprotokolls kann der virtuelle Tomograph im Kontext quantitativer Studien eingesetzt werden wie folgt:

An den beteiligten PET Systemen werden - nicht notwendigerweise zu Beginn der Studie - unter den Bedingungen des Untersuchungsprotokolls Phantom Messungen durchgeführt.

Diese Messungen werden in der beschriebenen Weise jeweils linear rekonstruiert und die jeweilgen PSF der beteiligten PET-Systeme bestimmt.

Wenn die verschiedenen PSF der beteiligten Systeme vorliegen kann eine geeignete räumliche Grenzfrequenz $\omega$ bestimmt werden und eine entsprechende standardisierte PSF für den virtuellen Tomographen gewählt werden, etwa in Form der in Gleichung (15) beschriebenen Funktion $F^{-1}(H_\omega)$ .

In der Folge kann für jedes der beteiligten PET-Systeme die oben beschriebene Transkonvolution-Funktion $ntf_\omega$ bestimmt werden.

Im Verlauf der multizentrischen Studie wird für jede Messung zusätzlich zu der üblichen iterativen Rekonstruktion eines Bilddatensatzes für optimierte Bildgebung eine zweite lineare Rekonstruktion berechnet und diese einer Konvolution mit der für den jeweiligen Tomographen bestimmten Transkonvolution-Funktion unterworfen. Der resultierende quantitativ normalisierte zweite Bilddatensatz repräsentiert die Messung, wie sie durch den virtuellen Tomographen durchgeführt worden wäre.

Beide Bilddatensätze können im Rahmen der üblichen Verfahren parallel gehandhabt, visualisiert, bewertet und gespeichert werden.

Die visuelle Begutachtung erfolgt anhand der iterativ rekonstruierten und für die Bildgebung optimierten Bilddatensätze, quantitative Auswertungen welcher Art auch immer erfolgen anhand der genannten, normalisierten Bilddatensätze. Die Ergebnisse dieser quantitativen Auswertungen sind dann auch für Messungen von unterschiedlichen Systemen vergleichbar.

**Literatur**

[BENE01]   Benedetto J. Modern sampling theory: mathematics and applications. Birhäuser Boston 2001, ISBN 0-8176-4023-1

[BOEL03]   Boellaard R, Krak NC, Hoekstra OS, Lammertsma AA. Effects of noise, image resolution, and ROI definition on the accuracy of standard uptake values: a simulation study. J Nucl Med. 2004;45:

1519-1527.

[BOEL09] Boellaard R. Standards for PET image acquisition and quantitative data analysis. J Nucl Med. 2009; 50(suppl 2):11S-20S

[BOEL09b] Boellaard R, et al. FDG PET and PET/CT: EANM procedure guidelines for tumour PET imaging: version 1.0, Eur J Nucl Med Mol Imaging, 2009, Nov.

[CHER06] Cherry SR. The 2006 Henry N. Wagner Lecture: Of Mice and Men (and Positrons) - Advances in PET Imaging Technology. J Nucl Med 47: 1735-1745.

[DAUB02] Daube-Witherspoon ME et al. PET Performance Measurements Using the NEMA NU 2-2001 Standard. J Nucl Med 2002; 43:1398-1409

[DELBE2006] Delbeke D, Coleman RE, Guiberteau MJ, et al. Procedure guideline for tumor imaging with 18F-FDG PET/CT 1.0. J Nucl Med. 2006;47:885-895

[ERSO07] Ersoy OK. Diffraction, Fourier Optics and Imaging. Wiley 2007, ISBN 978-0-471-23816-4

[GEWO02] Geworski L, Knoop BO et al. Multicenter comparison of calibration and cross calibration of PET scanners. J Nucl Med. 2002;43:635-639

[GOOD96] Goodman J. Introduction to Fourier Optics. McGraw Hill, 1996, ISBN

[HERR06] Herraiz JL et al. Optimal and Robust PET Data Sinogram Restoration Based on the Response of the System. IEEE 2006 Nuclear Science Symposium Conference Record p.3404-3407

[HERR07] Herraiz JL et al. Noise and physical limits to maximum resolution of PET images. Nuclear Instruments and Methods in Physics Research A 580 (2007) p.934-937

[KEYE95] Keyes JW. SUV: Standard Uptake or Silly Useless Value ?. J Nucl Med. 1995;36:1836-1839.

[OLDE74] Oldendorf WH. Expression of Tissue Isotope Distribution. J Nucl Med 15: 725-a-726-a.

[SCHE09] Scheuermann JS. Qualification of PET Scanners for Use in Multicenter Cancer Clinical Trials: The American College of Radiology Imaging Network Experience. J Nucl Med 2009 50: 1187-1193.

[SHYA08] Shyam SM et al. A recovery coefficient method for partial volume correction of PET images. Ann Nucl Med (2009) 23:341-348

[SORE07] Soret M et al. Partial-Volume Effect in PET Tumour Imaging. J Nucl Med. 2007;48:932-945

[STRA91] Strauss LG, Conti PS. The Applications of PET in Clinical Oncology. J Nucl Med 1991:32: 623-648.

[TARA03] Tarantola G et al. PET Instrumentation and Reconstruction Algorithms in Whole-Body Applications. J Nucl Med 44: 756-769.

[TOWN08] Townsend DW. Dual-Modality Imaging: Combining Anatomy and Function. J Nucl Med. 2008;49: 938-955

[WEST06] Westerterp M et. al., Quantification of FDG PET studies using standardised uptake values in multicentre trials: effects of image reconstruction, resolution and ROI definition parameters. Eur J Nucl Med Mol Imaging. 2007 Mar;34(3):392-404. Epub 2006 Oct 11.

[WOOD75] Woodard HQ et al. Expression of Tissue Isotope Distribution. J Nucl Med 16: 958-b-959-b.

## Patentansprüche

1. Verfahren zur einheitlichen und vergleichbaren Bestimmung normalisierter quantitativer Messwerte ausgehend von Bildern bzw. Bilddaten, welche von unterschiedlichen bildgebenden Systemen aufgenommen wurden, **gekennzeichnet durch** folgende Verfahrensschritte:

a) Für jedes der genannten bildgebenden Systeme eine Festlegung der für die Aufnahme der genannten Bilder bzw. Bilddaten zu verwendenden Aufnahmeparameter dergestalt, dass eine im Rahmen der Möglichkeiten der genannten Systeme möglichst lineare Bildaufnahme erfolgt;

b) Für jedes der genannten bildgebenden Systeme unter Verwendung der genannten Aufnahmeparameter eine Messung oder anderweitige Bestimmung einer stationären oder nicht stationären räumlichen Transferfunktion ( $tf$ ), welche die Aufnahmeeigenschaften des jeweiligen Systems beschreibt;

c) Die Festlegung einer stationären oder nicht stationären räumlichen Transferfunktion ( $ntf$ ), welche die Aufnahmeeigenschaften eines virtuellen normalisierten bildgebenden Systems beschreibt;

d) Die numerische Berechnung normalisierter Bilder bzw. Bilddaten ( $n$ ) ausgehend von den **durch** die genannten bildgebenden Systeme aufgenommenen Bildern bzw. Bilddaten ( $d$ ), welche eine Transkonvolution umfasst, wobei die Transkonvolution dabei darstellbar ist als eine Konvolution der jeweiligen aufgenommenen Bilder bzw. Bilddaten ( $d$ ) mit dem Ergebnis einer Konvolution der Inversen der genannten räumlichen Transferfunktion des jeweiligen bildgebenden Systems ( $tf^{-1}$ ) mit der genannten festgelegten stationären oder nicht stationären räumlichen Transferfunktion ( $ntf$ ) des virtuellen normalisierten bildgebenden Systems:

$$d \otimes tf^{-1} \otimes ntf = n \ ;$$

und

e) Die Bestimmung der genannten normalisierter quantitativer Messwerte anhand der den genannten aufgenommenen Bildern bzw. Bilddaten entsprechenden genannten berechneten normalisierten Bilder bzw. Bilddaten.

2. Das Verfahren gemäss Anspruch 1 **gekennzeichnet dadurch, dass** die Bilddaten 2-dimensional sind und eine Messung in 2 räumlichen Dimensionen repräsentieren, oder, dass die Bilddaten 3-dimensional sind und eine Messung in 3 räumlichen Dimensionen repräsentieren,

3. Das Verfahren gemäss Anspruch 1 **gekennzeichnet dadurch, dass** die Bilddaten neben 2 oder 3 räumlichen Dimensionen eine oder mehrere weitere nicht räumliche Dimensionen umfassen und das genannte Verfahren nur bezüglich der räumlichen Dimensionen durchgeführt wird.

4. Ein Verfahren gemäss einem der Ansprüche 1 bis 3 **gekennzeichnet dadurch, dass** die genannten bildgebenden Systeme tomographische Systeme sind und die genannten Bilddatensätze Sinogramme.

5. Ein Verfahren gemäss einem der Ansprüche 1 bis 3 **gekennzeichnet dadurch, dass** die genannten bildgebenden Systeme tomographische Systeme sind und die genannten Bilddatensätze rekonstruierte Bilder.

6. Ein Verfahren gemäss einem der Ansprüche 4 oder 5 **gekennzeichnet dadurch, dass** die genannten tomographischen Systeme Emissionstomographen sind und über eine Möglichkeit zur Dämpfungskorrektur verfügen.

7. Ein Verfahren gemäss Anspruch 6 **gekennzeichnet dadurch, dass** die genannten Emissionstomographen PET/CT oder SPECT/CT Systeme sind.

8. Ein Verfahren gemäss einem der vorangehenden Ansprüche **gekennzeichnet dadurch, dass** die genannte Messung einer stationären oder nicht stationären räumlichen Transferfunktion ( *tf* ), welche die Aufnahmeeigenschaften des jeweiligen Systems beschreibt, die Verwendung eines Phantoms umfasst..

9. Ein Verfahren gemäss Anspruch 8 **gekennzeichnet dadurch, dass** das genannte Phantom als Festkörper ausgebildete Strahlungsquellen von definierter Grösse, Form und Aktivität enthält und für die genannte Messung einer stationären oder nicht stationären räumlichen Transferfunktion mehrfach verwendet werden kann.

10. Ein Verfahren gemäss Anspruch 9 **gekennzeichnet dadurch, dass** die Bestimmung einer stationären oder nicht stationären räumlichen Transferfunktion ( *tf* ), welche die Aufnahmeeigenschaften des jeweiligen Systems bestimmt, neben der genannten Messung unter Verwendung des genannten Phantoms eine numerische Interpolation oder Extrapolation der nichtstationären Transferfunktion entsprechend bekannten Eigenschaften des jeweiligen tomographischen Systems umfasst.

**Claims**

1. A method for standardized and comparable determination of normalized quantitative measurement values based on images or image data acquired by different imaging systems, **characterized by** the following method steps:

a) for each of said imaging systems a determination of acquisition parameters to be used to acquire said images or image data such that within the capabilities of said systems a most linear image acquisition is performed;
b) for each of said imaging systems using said acquisition parameters a measurement or other determination of a stationary or non-stationary spatial transfer function (tf), which describes the imaging properties of the respective system;
c) definition of a stationary or non-stationary spatial transfer function (ntf), which describes the imaging properties of a virtual normalized imaging system;

d) numerical calculation of normalized images or image data (***n***) starting from images or image data (***d***) acquired by said imaging systems comprising Transconvolution, where said Transconvolution is representable as a convolution of the respective acquired images or image data (***d***) with the result of a convolution of the inverse of said spatial transfer function of the respective imaging system (***tf-1***) with said defined stationary or non-stationary spatial transfer function (***ntf***) of the virtual normalized imaging system:

$$d \otimes tf^{-1} \otimes ntf = n \, ;$$

and

e) determination of said normalized quantitative measurement values using said calculated normalized images or image data which correspond to said acquired images or image data.

2. A method according to claim 1 **characterized in that**
the image data are two-dimensional and represent a measurement in two spatial dimensions, or that
the image data are three-dimensional and represent a measurement in three spatial dimensions.

3. A method according to claim 1, **characterized in that**
the image data in addition to either two or three spatial dimensions comprise one or more additional non-spatial dimensions and said method is carried out only with respect to the spatial dimensions.

4. A method according to one or more of claims 1 to 3, **characterized in that** said imaging systems are tomographic systems and said image data sets are sinograms.

5. A method according to one of claims 1 to 3, **characterized in that** said imaging systems are tomographic systems and said image data sets are reconstructed images.

6. A method according to one of claims 4 or 5, **characterized in that** said tomographic systems are emission tomographic systems and are able to perform attenuation correction.

7. A method according to claim 6, **characterized in that** said emission tomographic systems are PET / CT or SPECT / CT systems.

8. A method according to one of the preceding claims **characterized in that** said measurement of a stationary or non-stationary spatial transfer function (***tf***), which describes the imaging properties of the respective system, involves the use of a phantom.

9. A method according to claim 8, **characterized in that** said phantom contains solid state radiation sources of defined size, shape and activity, and can be used repeatedly for said measurement of a stationary or non-stationary spatial transfer function.

10. A method according to claim 9, **characterized in that** the determination of a stationary or non-stationary spatial transfer function (***tf***), which describes the imaging properties of the respective system, comprises a numerical interpolation or extrapolation of the non-stationary transfer function in accordance with known properties of the respective tomographic system in addition to said measurement using said phantom.

**Revendications**

1. Procédé destiné à la détermination uniforme et comparable de valeurs de mesure quantitatives normalisées en partant d'images et/ou de fichiers d'images qui ont été enregistrés par différents systèmes transmetteurs d'images, **caractérisé par** les étapes de procédé suivantes :

a) Pour chacun desdits systèmes transmetteurs d'images, une spécification des paramètres d'enregistrement à utiliser pour l'enregistrement desdits images et/ou fichiers d'images, de sorte qu'un enregistrement d'images, si possible linéaire, s'effectue dans le cadre des possibilités des systèmes nommés ;
b) Pour chacun desdits systèmes transmetteurs d'images, en utilisant lesdits paramètres d'enregistrement, une mesure ou autre détermination d'une fonction de transfert spatial stationnaire ou non stationnaire (*tf*) qui décrit les propriétés d'enregistrement du système respectif ;

c) La spécification d'une fonction de transfert spatial stationnaire ou non stationnaire (*ntf*) qui décrit les propriétés d'enregistrement d'un système virtuel normalisé transmetteur d'images ;

d) Le calcul numérique d'images et/ou de fichiers d'images normalisés (*n*) en partant des images et/ou fichiers d'images (*d*) enregistrés par lesdits systèmes transmetteurs d'images, qui comprend une transconvolution, la transconvolution étant alors représentable comme une convolution des images et/ou fichiers d'images (*d*) enregistrés respectifs par le résultat d'une convolution des inverses de ladite fonction de transfert spatial du système transmetteur d'images (*tf¹*) respectif avec ladite fonction de transfert spatial stationnaire ou non stationnaire (*ntf*) du système transmetteur d'images virtuel normalisé :

$$d \otimes tf^{-1} \otimes ntf = n \; ;$$

et

e) La détermination desdites valeurs de mesure quantitatives normalisées à l'aide desdits images et/ou fichiers d'images normalisés calculés correspondant aux-dits images et/ou fichiers d'images enregistrés.

2. Procédé selon la revendication 1, **caractérisé**
**en ce que** les fichiers d'image sont à 2 dimensions et représentent une mesure dans 2 dimensions spatiales, ou **en ce que** les fichiers d'image sont à 3 dimensions et représentent une mesure dans 3 dimensions spatiales.

3. Procédé selon la revendication 1, **caractérisé**
**en ce que** les fichiers d'image, outre 2 ou 3 dimensions spatiales, comprennent une ou plusieurs autres dimensions non spatiales et en ce que ledit procédé est uniquement exécuté par rapport aux dimensions spatiales.

4. Procédé selon une quelconque des revendications 1 à 3, **caractérisé en ce que** lesdits systèmes transmetteurs d'images sont des systèmes tomographiques et lesdits enregistrements d'images des sinogrammes.

5. Procédé selon une quelconque des revendications 1 à 3, **caractérisé en ce que** lesdits systèmes transmetteurs d'images sont des systèmes tomographiques et lesdits enregistrements d'images des images reconstruites.

6. Procédé selon une quelconque des revendications 4 ou 5, **caractérisé en ce que** lesdits systèmes tomographiques sont des tomographes d'émission et disposent d'une possibilité pour la correction d'atténuation.

7. Procédé selon la revendication 6, **caractérisé**
**en ce que** lesdits tomographes d'émission sont des systèmes PET/CT ou SPECT/CT.

8. Procédé selon une quelconque des revendications précédentes, **caractérisé en ce que** ladite mesure d'une fonction de transfert spatial stationnaire ou non stationnaire (*tf*), qui décrit les propriétés d'enregistrement du système respectif, comprend l'utilisation d'un fantôme.

9. Procédé selon la revendication 8, **caractérisé**
**en ce que** ledit fantôme contient en tant que corps solide des sources de rayonnement développées de taille, de forme et d'activité définie et peut être utilisé plusieurs fois pour ladite mesure d'une fonction de transfert spatial stationnaire ou non stationnaire.

10. Procédé selon la revendication 9, **caractérisé**
**en ce que** la détermination d'une fonction de transfert spatial stationnaire ou non stationnaire (*tf*) qui détermine les propriétés d'enregistrement du système respectif, comprend, outre ladite mesure en utilisant ledit fantôme, une interpolation ou une extrapolation numérique de la fonction de transfert non stationnaire en fonction des propriétés connues du système tomographique respectif.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Partial Volume Correction Strategies in PET. **OLIVER ROUSSET et al.** PET Clin. Elsevier Saunders, 2007, vol. 2, 235-249
- **BENEDETTO J.** Modern sampling theory: mathematics and applications. *Birhäuser Boston,* 2001, ISBN 0-8176-4023-1
- **BOELLAARD R ; KRAK NC ; HOEKSTRA OS ; LAMMERTSMA AA.** Effects of noise, image resolution, and ROI definition on the accuracy of standard uptake values: a simulation study. *J Nucl Med.,* 2004, vol. 45, 1519-1527
- **BOELLAARD R.** Standards for PET image acquisition and quantitative data analysis. *J Nucl Med.,* 2009, vol. 50 (2), 11S-20S
- **BOELLAARD R et al.** FDG PET and PET/CT: EANM procedure guidelines for tumour PET imaging: version 1.0. *Eur J Nucl Med Mol Imaging,* November 2009
- **CHERRY SR.** The 2006 Henry N. Wagner Lecture: Of Mice and Men (and Positrons) - Advances in PET Imaging Technology. *J Nucl Med,* 2006, vol. 47, 1735-1745
- **DAUBE-WITHERSPOON ME et al.** PET Performance Measurements Using the NEMA NU 2-2001 Standard. *J Nucl Med,* 2002, vol. 43, 1398-1409
- **DELBEKE D ; COLEMAN RE ; GUIBERTEAU MJ et al.** Procedure guideline for tumor imaging with F-FDG PET/CT 1.0. *J Nucl Med.,* 2006, vol. 47, 885-895
- **ERSOY OK.** Diffraction, Fourier Optics and Imaging. Wiley, 2007
- **GEWORSKI L ; KNOOP BO et al.** Multicenter comparison of calibration and cross calibration of PET scanners. *J Nucl Med.,* 2002, vol. 43, 635-639
- **GOODMAN J.** Introduction to Fourier Optics. McGraw Hill, 1996
- **HERRAIZ JL et al.** Optimal and Robust PET Data Sinogram Restoration Based on the Response of the System. *IEEE 2006 Nuclear Science Symposium Conference Record,* 2006, 3404-3407
- **HERRAIZ JL et al.** Noise and physical limits to maximum resolution of PET images. *Nuclear Instruments and Methods in Physics Research A,* 2007, vol. 580, 934-937
- **KEYES JW.** SUV: Standard Uptake or Silly Useless Value ?. *J Nucl Med.,* 1995, vol. 36, 1836-1839
- **OLDENDORF WH.** Expression of Tissue Isotope Distribution. *J Nucl Med,* vol. 15, 725-726
- **SCHEUERMANN JS.** Qualification of PET Scanners for Use in Multicenter Cancer Clinical Trials: The American College of Radiology Imaging Network Experience. *J Nucl Med,* 2009, vol. 50, 1187-1193
- **SHYAM SM et al.** A recovery coefficient method for partial volume correction of PET images. *Ann Nucl Med,* 2009, vol. 23, 341-348
- **SORET M et al.** Partial-Volume Effect in PET Tumour Imaging. *J Nucl Med.,* 2007, vol. 48, 932-945
- **STRAUSS LG ; CONTI PS.** The Applications of PET in Clinical Oncology. *J Nucl Med,* 1991, vol. 32, 623-648
- **TARANTOLA G et al.** PET Instrumentation and Reconstruction Algorithms in Whole-Body Applications. *J Nucl Med,* vol. 44, 756-769
- **TOWNSEND DW.** Dual-Modality Imaging: Combining Anatomy and Function. *J Nucl Med.,* 2008, vol. 49, 938-955
- **WESTERTERP M.** Quantification of FDG PET studies using standardised uptake values in multi-centre trials: effects of image reconstruction, resolution and ROI definition parameters. *Eur J Nucl Med Mol Imaging. 2007 Mar,* 11. Oktober 2006, vol. 34 (3), 392-404
- **WOODARD HQ et al.** Expression of Tissue Isotope Distribution. *J Nucl Med,* vol. 16, 958-959